Europäisches Patentamt

European Patent Office

Office européen des brevets

(11) Veröffentlichungsnummer: **0 312 695 B1**

# EUROPÄISCHE PATENTSCHRIFT

(12)

(45) Veröffentlichungstag der Patentschrift:
**14.11.90**

(51) Int. Cl.⁵: **A61F 13/02**

(21) Anmeldenummer: **88109579.8**

(22) Anmeldetag: **16.06.88**

(54) Selbstklebendes Wundnahtpflaster.

(30) Priorität: **19.03.88 DE 3809348**
**23.10.87 DE 3735894**

(43) Veröffentlichungstag der Anmeldung:
**26.04.89 Patentblatt 89/17**

(45) Bekanntmachung des Hinweises auf die Patenterteilung:
**14.11.90 Patentblatt 90/46**

(84) Benannte Vertragsstaaten:
**AT BE CH DE ES FR GB GR IT LI NL SE**

(56) Entgegenhaltungen:
**EP-A- 0 028 452**
**EP-A- 0 230 373**
**DE-A- 2 733 549**
**DE-A- 3 524 315**
**DE-B- 1 164 021**
**GB-A- 2 081 177**

**Die Akte enthält technische Angaben, die nach dem Eingang der Anmeldung eingereicht wurden und die nicht in dieser Patentschrift enthalten sind.**

(73) Patentinhaber: **Beiersdorf Aktiengesellschaft, Unnastrasse 48, D-2000 Hamburg 20(DE)**

(72) Erfinder: **Heimerl, Albert, Dr., Immenhorstweg 70, D-2000 Hamburg 65(DE)**
Erfinder: **Schulte, Dietrich, Dr., Reichenberger Strasse 11, D-2080 Pinneberg(DE)**
Erfinder: **Götz, Gabriela, Im Saseler Mühlenweg 27, D-2000 Hamburg 65(DE)**
Erfinder: **Leutz, Reiner, Tannenallee 47, D-2057 Reinbek(DE)**

ACTORUM AG

**Beschreibung**

Die Erfindung betrifft ein selbstklebendes Wundnahtpflaster mit einem elastischen Trägermaterial.

Wundnahtpflaster werden zum Verschluß oder zur Fixierung von Hautläsionen verwendet. Sie bestehen im allgemeinen aus einem streifenförmigen Trägermaterial, das ganz oder teilweise mit einer Klebemasse beschichtet ist und werden bei ihrer Anwendung quer über die Wunde geklebt, um die Wundränder zusammenzuhalten. Dadurch soll erreicht werden, daß im Gegensatz zu genähten Wundrändern beim Abheilen kaum sichtbare Narben zurückbleiben.

Als Trägermaterialien für derartige Wundnahtpflaster wurden bisher schon die verschiedensten Gewebe, Vliese oder Folien vorgeschlagen bzw. eingesetzt. Wobei diese Materialien z. Teil einerseits zwar anschmiegsam und gut luft- und wasserdampfdurchlässig aber unnachgiebig sind, wie beispielsweise gemäß DE-Gbm 70 32 197, und z. Teil andererseits elastisch und dem Wundheilungsprozeß sehr nachgebend ausgebildet sind, wie beispielsweise gemäß EP-OS 28 452, EP-OS 230 373 und DE-OS 35 24 315.

Diese elastischen Wundverschlußstreifen aus Polyurethanfolie, geprägtem Vlies oder in einem Winkel zur Hauptstreckachse geschnittenem Polymermaterial geben zwar beim Heilungsprozeß einer Wunde, der in der Regel mit einem mehr oder weniger starken Anschwellen des Gewebes durch Ödembildung gekennzeichnet ist, plastisch nach und verhindern so eine Schädigung des Gewebes durch zu starkes Zusammenpressen, sie weisen jedoch eine zu geringe Spannkraft auf, um einen optimalen Wundverschluß zu gewährleisten. Nach dem Abklingen der Schwellung bildet sich aufgrund der plastischen Ausdehnung dieser Produkte über dem Wundspaltbereich in der Regel eine unerwünscht breite Narbe aus.

Wundnahtstreifen gemäß der EP-A 28 452, die vorzugsweise aus einer Polyurethan-Folie bestehen, sollen sich zwar elastisch und rückstellfähig verhalten, die dort beschriebenen Dehnungswerte von wenigstens 8%, bevorzugt wenigstens 20%, ganz bevorzugt 40%, bei einer Belastung von nur 120–160 g/cm, sind jedoch viel zu hoch, d.h., die Pflaster sind entsprechend zu nachgiebig und ergeben deshalb ebenfalls keine einwandfreie Narbe mit dicht aneinanderliegenden Wundrändern.

Aufgabe der Erfindung war es, ein Wundnahtpflaster zu entwickeln, das sich dem physiologischen Heilungsprozeß einer Narbe vollkommen anpaßt und zu jedem Zeitpunkt dieses Vorgangs ein spannungsfreies aber festes Zusammenhalten der Wundränder gewährleistet.

Gelöst wird diese Aufgabe durch einen Wundnahtstreifen mit einem elastischen Trägermaterial gemäß Anspruch 1.

Das bedeutet, dar er zwar beim Anschwellen des Gewebes ausreichend elastisch nachgibt, aber dem für den fortschreitenden Heilungsprozeß üblichen Abschwellen aufgrund seiner reversiblen Dehnungsfähigkeit ebenfalls wieder folgt.

Bevorzugt ist diese reversible Elastizität, die sich bei der Messung als eine auf ihren Ausgangspunkt wieder ganz oder zumindest beinahe ganz zurückführende Hysteresekurve darstellt, derart eingestellt, daß der Wundnahtstreifen bei einer Zugkrafteinwirkung von 5 N/cm eine Längenausdehnung zwischen etwa 1 und 15%, bei 10 N/cm eine Längenausdehnung zwischen etwa 2 und 20% und bei 20 N/cm eine Längenausdehnung zwischen etwa 6 und 35% aufweist.

Als besonders geeignet haben sich Produkte erwiesen mit einer Ausdehnung zwischen etwa 2 und 10% bei 5 N/cm, zwischen etwa 5 und 15% bei 10 N/cm und zwischen etwa 15 und 30% bei 20 N/cm Breite (gemessen an einer üblichen Zugprüfmaschine bei verschiedenen Zuggeschwindigkeiten (zwischen 20 und 300 mm/min) und Streifenbreiten von 0,4 bis 2,6 cm Breite). Derartige Dehnungswerte liegen deutlich unterhalb der Dehnungswerte von elastischen Binden, Bandagen und auch Pflastern, wie sie üblicherweise in der medizinischen Praxis verwendet werden. Die Werte sind sogar so gering, daß das Material beim einfachen Zugversuch von Hand kaum elastisch wirkt. Sie sind jedoch den natürlichen Schwellbewegungen der Wunde angepaßt.

Bei einer stark anschwellenden Wunde - beispielsweise durch eine Entzündung hervorgerufen - empfiehlt es sich, ein etwas dehnbareres Pflaster einzusetzen als bei einer Wunde, die weniger Schwellung zeigt, um einen optimalen Wundverschluß zu erreichen.

Obwohl im Prinzip entsprechende Gewirke, Gewebe, Vliese oder Folien als Trägermaterial geeignet sind, wird bevorzugt ein Gewirke verwendet.

Derartige elastische Gewirke können beispielsweise aus Polyamid-, Polyester- oder Polyurethan/Zellwoll-Fäden bestehen Bevorzugt wird ein Polyamid-Gewirke mit hoher Reißfestigkeit

Das Trägermaterial ist in bekannter Weise mit einer mikroporösen Selbstklebeschicht versehen, um eine gute Luft- und Wasserdampfdurchlässigkeit zu gewährleisten. Wird das bevorzugte Gewirke eingesetzt, ergeben sich aufgrund der vergleichsweise großen Poren im Träger besonders gute Werte, nämlich eine Luftdurchlässigkeit von mindestens 10 cm³/cm²sec und eine Wasserdampfdurchlässigkeit von mindestens 700 g/m². Als gut hautverträgliche Klebemassen mit hoher Anfaßklebrigkeit haben sich solche aus Polyacrylsäureestern oder Acrylsäureester-Copolymerisaten, insbesondere gemäß DE-PS 27 43 979, bewährt.

Bei der technischen Ausführung zur Herstellung der erfindungsgemäßen Wundnahtpflaster wird die Klebemasse, vorzugsweise als Lösung, auf einen klebstoffabweisend ausgerüsteten Zwischenträger, beispielsweise silikonisiertes Kraftpapier, ausgestrichen und durch schnelles Verdampfen des Lösungsmittels bei erhöhter Temperatur zu einem feinblasigen Zustand getrocknet. Nach Abkühlen auf Raumtemperatur wird das eigentliche Trägermaterial auf die Klebeschicht aufkaschiert und gegebenenfalls einem Kalandervorgang gemäß DBP 15 69 901 unterworfen. Dadurch wird die Klebemasse auf das Trägermaterial übertragen und die feinen Bläschen werden aufgebrochen, wodurch ein feinporiges mikroporöses Gefüge entsteht. Wenn es die Klebemasse erfordert, kann noch ein Vernetzungsprozeß durch Wärmeeinwirkung oder UV-Bestrahlung nachgeschaltet werden.

Anschließend wird das Produkt für den Gebrauch konfektioniert, d.h in Streifen gewünschter Länge und Breite geschnitten, auf den endgültigen Hilfsträger mit Abständen zwischen den einzelnen Streifen umkaschiert, mikrobiologisch dicht eingeschweißt und durch γ-Bestrahlung sterilisiert.

Die auf diese Weise hergestellten, erfindungsgemäßen Wundnahtpflaster mit einem reversibel elastischen Trägermaterial erfüllen alle von der Praxis an sie gestellte Forderungen. Sie sind so geschmeidig und flexibel, daß sie sich den Unebenheiten der Haut anpassen, aber gleichzeitig so dehnbar, rückstellfähig und reißfest, daß die Wundränder ohne Gewebeschädigung zu jedem Zeitpunkt fest zusammengehalten werden. Dies bedeutet eine zuverlässige Adaptierung der Wundränder, bis die Narbe genügend Eigenfestigkeit aufgebaut hat.

Die folgenden Beispiele sollen die Herstellung der neuen Wundnahtpflaster ausführlicher erläutern:

## Beispiel 1

Auf einem silikonisierten Trennpapier wird eine Selbstklebemasse, die durch Copolymerisation von 49 Gewichtsteilen 2-Äthylhexylacrylat, 49 Gewichtsteilen n-Butylacrylat und 2 Gewichtsteilen Glycidylmethacrylat erhalten wurde, aus einer Lösung in einem Aceton-Benzin-Gemisch in einer solchen Menge aufgetragen, daß nach dem Trocknen eine Schichtstärke von etwa 50 g/m² erzielt wird Um das Lösungsmittelgemisch rasch abzudunsten, wird das mit der Klebemasse beschichtete Trennpapier anschließend durch einen stufenweise beheizten Trockenkanal mit Temperaturen von 60 - 100°C geleitet, wodurch sich in der Klebeschicht eine Vielzahl winziger Bläschen bildet. Nach Abkühlung auf Raumtemperatur wird auf die getrocknete Klebeschicht ein Polyamid-6,6-Gewirke mit folgenden Daten aufkaschiert:

|  | Stäbchen | Reihen |
|---|---|---|
| Fadendichte (Faden/cm): | ca. 12 | ca. 21 |
| Garnstärke (dtex): | 44 | 44 |
| Reißkraft (N/cm): | ca. 55 | ca. 130 |
| Reißdehnung (%): | ca. 50 | ca. 70 |
| Flächengewicht: ca. 75 g/m² | | |

Danach wird das Verbundprodukt bei einem Druck von etwa 5 kp/cm² kalandert Nach diesem Kalandervorgang, bei dem die kleinen Bläschen aufbrechen und gleichzeitig die Klebeschicht sich fest mit dem Gewirke verbindet, werden die breiten Rollen zu schmalen Rollen unterschiedlicher Breiten aufgeschnitten, wird das noch anhaftende Trennpapier abgezogen, und die geschnittenen Bänder werden in Abschnitten von je 10 cm Länge zu mehreren nebeneinander erneut auf ein silikonisiertes Trennpapier aufgelegt. Nach dem Verpacken werden die fertigen Wundnahtpflaster einer γ-Bestrahlung (2,5 Mrad absorbierte Dosis) unterworfen und auf diese Weise sterilisiert. Gleichzeitig erfährt die Klebemasse eine Nachvernetzung, die ihre Kohäsion in wünschenswerter Weise verbessert.

## Beispiel 2

Ein Polyamidgewirke-Trägermaterial, wie in Beispiel 1 beschrieben, wird mit einer UV-vernetzbaren Selbstklebemassenschicht gemäß DBP 27 43 979 versehen. Dabei wird im wesentlichen verfahren wie im vorstehenden Beispiel 1 ausgeführt, jedoch ein Copolymerisat aus 73,59 Gew -%. 2-Äthylhexylacrylat, 20 Gew.-% Butylacrylat, 6 Gew -% Acrylsäure und 0,41 Gew.-% Benzoinacrylat eingesetzt.

Nach dem Zusammenkaschieren des die getrocknete Selbstklebeschicht tragenden Trennpapiers mit dem Gewebeträger wird das Verbundprodukt von der Gewebeseite her etwa 1 sec lang mit 4 x 11-12 kW UV-Strahlung bestrahlt, um die Klebemasse zu vernetzen. Die Weiterverarbeitung und Konfektionierung erfolgt analog Beispiel 1. Das Produkt zeigt eine besonders gute Anfaßklebrigkeit (tack).

Die Dehnungsmessungen mit diesem Produkt an einer Zugprüfmaschine ergab bei Zugkräften von 5, 10 und 20 N/cm folgende Werte:
bei 5 N/cm zwischen etwa 4 und 10%,
bei 10 N/cm zwischen etwa 6 und 14%,
bei 20 N/cm zwischen etwa 17 und 30%, gemessen an unterschiedlich breiten Streifen (0,4 - 2,6 cm) verschiedener Materialchargen, bei unterschiedlichen Zuggeschwindigkeiten (20, 100 und 300 mm/min) sowie unterschiedlichen Einspannlängen (20, 50, 60 und 100 mm). Die Dehnung war praktisch jedes Mal reversibel, d.h. nach Zugentlastung nahmen die Streifen wieder die ursprüngliche Länge an Vereinzelte geringfügige Restwerte ergeben sich u.a aus der nie vollkommenen Homogenität des Materials.

**Patentansprüche**

1. Selbstzklebendes Wundnahtpflaster, insbesondere in Streifenform, mit einem elastischen Trägermaterial, wobei sich der Trägerstreifen unter Zugkrafteinwirkung ausdehnt und sich nach Beendigung dieser Krafteinwirkung praktisch wieder vollständig auf die ursprüngliche Länge zurückstellt, dadurch gekennzeichnet, daß der Trägerstreifen bei einer Zugkrafteinwirkung von 5N/cm eine Längenausdehnung zwischen etwa 1 und 15% aufweist.

2. Wundnahtpflaster gemäß Anspruch 1, dadurch gekennzeichnet, daß der Trägerstreifen bei einer Zugkrafteinwirkung von 10 N/cm eine Längenausdehnung zwischen etwa 2 und 20% und bei 20 N/cm eine Längenausdehnung zwischen etwa 6 und 35% aufweist.

3. Wundnahtpflaster gemäß Anspruch 1, dadurch gekennzeichnet, daß der Trägerstreifen bei einer Zugkrafteinwirkung von 5 N/cm eine Längenausdehnung zwischen etwa 2 und 10%, bei 10 N/cm eine Längenausdehnung zwischen etwa 5 und 15% und bei 20 N/cm eine Längenausdehnung zwischen etwa 15 und 30% aufweist.

4. Wundnahtpflaster gemäß Anspruch 1, dadurch gekennzeichnet, daß das Trägermaterial aus einem elastischen Gewirke besteht.

5. Wundnahtpflaster gemäß Anspruch 1, dadurch gekennzeichnet, daß das Trägermaterial aus einem elastischen Polyamid-Gewirke besteht.

6. Wundhahtpflaster gemäß Ansprüch 1, dadurch gekennzeichnet, daß es eine Luftdurchlässigkeit von mindestens 10 cm³/cm²sec und eine Wasserdampfdurchlässigkeit von mindestens 700 g/m² aufweist.

7. Verwendung eines selbstklebenden Pflasters gemäß einem oder mehreren der Ansprüche 1 bis 6 zur Herstellung eines Wundnahtpflasters.

**Claims**

1. Self-adhesive wound suture plaster, especially in strip form, with an elastic carrier material, in which the carrier strip extends under the action of a pulling force and, after this action of force has ended, virtually returns completely to the original length, characterized in that the carrier strip undergoes a length extension of between about 1 and 15% under the action of a pulling force of 5 N/cm.

2. Wound suture plaster according to Claim 1, characterized in that the carrier strip undergoes a length extension of between about 2 and 20% under the action of a pulling force of 10 N/cm and a length extension of between about 6 and 35% at 20 N/cm.

3. Wound suture plaster according to Claim 1, characterized in that the carrier strip undergoes a length extension of between about 2 and 10% under the action of a pulling force of 5 N/cm, a length extension of between about 5 and 15% at 10 N/cm and a length extension of between about 15 and 30% at 20 N/cm.

4. Wound suture plaster according to Claim 1, characterized in that the carrier material consists of an elastic knitted fabric.

5. Wound suture plaster according to Claim 1, characterized in that the carrier material consists of an elastic knitted nylon fabric.

6. Wound suture plaster according to Claim 1, characterized in that it has an air permeability of at least 10 cm³/cm² sec and a water vapour permeability of at least 700 g/m².

7. Use of a self-adhesive plaster according to one or more of Claims 1 to 6 for producing a wound suture plaster.

**Revendications**

1. Emplâtre auto-adhésif pour suture de blessures, en particulier en forme de bande, avec un matériau de support élastique, dans lequel la bande de support s'allonge sous l'effet d'un effort de traction et en pratique revient complètement à sa longueur initiale lorsque cesse cet effort de traction, caractérisé en ce que la bande de support présente un allongement longitudinal compris entre environ 1 et 15% pour un effort de traction de 5 N/cm.

2. Emplâtre auto-adhésif suivant la revendication 1, caractérisé en ce que la bande de support présente un allongement longitudinal compris entre environ 2 et 20% pour un effort de traction de 10 N/cm et un allongement longitudinal compris entre environ 6 et 35% pour un effort de traction de 20 N/cm.

3. Emplâtre auto-adhésif suivant la revendication 1, caractérisé en ce que la bande de support présente pour un effort de traction de 5 N/cm un allongement longitudinal compris entre environ 2 et 10%, pour un effort de traction de 10 N/cm un allongement longitudinal compris entre environ 5 et 15% et pour un effort de traction de 20 N/cm un allongement longitudinal compris entre environ 15 et 30%.

4. Emplâtre auto-adhésif suivant la revendication 1, caractérisé en ce que le matériau de support est composé d'un tricot élastique.

5. Emplâtre auto-adhésif suivant la revendication 1, caractérisé en ce que le matériau de support est composé d'un tricot de polyamide élastique.

6. Emplâtre auto-adhésif suivant la revendication 1, caractérisé en ce qu'il présente une perméabilité à l'air d'au moins 10 $cm^3/cm^2s$ et une perméabilité à la vapeur d'eau d'au moins 700 $g/m^2$.

7. Utilisation d'un emplâtre auto-adhésif suivant l'une ou l'autre des revendications 1 à 6 pour la fabrication d'un emplâtre pour suture de blessures.